(19)

Europäisches
Patentamt
European
Patent Office
Office européen
des brevets

(11) **EP 4 261 834 A1**

(12) **EUROPEAN PATENT APPLICATION**

(43) Date of publication:
**18.10.2023 Bulletin 2023/42**

(21) Application number: **22168430.1**

(22) Date of filing: **14.04.2022**

(51) International Patent Classification (IPC):
**G16H 20/17** *(2018.01)*    **G16H 50/30** *(2018.01)*

(52) Cooperative Patent Classification (CPC):
**G16H 20/17; G16H 50/30**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **BIOTRONIK SE & Co. KG
12359 Berlin (DE)**

(72) Inventor: **Guhaniyogi, Shayan
Portland, 97206 (US)**

(74) Representative: **Biotronik Corporate Services SE
Corporate Intellectual Property
Sieversufer 7-9
12359 Berlin (DE)**

(54) **SYSTEM FOR SUPPORTING A PATIENT'S HEALTH CONTROL AND OPERATING METHOD OF SUCH SYSTEM**

(57)    The invention relates to a system (100) for supporting a patient's (110) health control comprising a medical device (140) and an extracorporeally located remote device (120), wherein the system has a training state and an application state, wherein the remote device comprises an artificial intelligence module (AI module, 121), wherein the remote device (120) and the medical device (140) are configured to be temporarily connected for data exchange using a communication connection, wherein in the application state the medical device (140) or the remote device (120) is configured to provide a suitable quality-of-life (QOL) recommendation (180) to the patient (110), wherein the suitable QOL recommendation (180) is determined based on at least one recently measured physiological data (160) of the patient by the medical device (140) and based on a QOL calculation specification provided by the AI module (121). The invention further relates to a corresponding operating method of such system, a respective computer program product and a respective computer readable data carrier.

FIG. 1

EP 4 261 834 A1

## Description

The invention is directed to a system for supporting a patient's health control comprising a medical device and an extracorporeally located remote device and to a respective operating method of such system method as well as a to computer program product and to a computer readable data carrier. The computer program product may be a software routine, e.g. related to hardware support means within the remote device and/or the medical device.

It is known that data related to a patient's health are collected by implanted and/or extracorporeally located medical devices and analyzed in a remote monitoring system to adapt the patient's therapy. Often these data are produced by specific physiological sensors (e.g. ECG, impedance, activity, posture, heart sounds, pressure, respiration ...) which are cost intensive and contained in complicated medical devices. To receive precise and locally identifiable results of such data, in many cases, the medical device needs to be implanted into the patient's body. An implanted medical device may, however, impair the patient's organism. Examples of such medical devices are a pacemaker (with leads), an implantable cardiac monitor (ICM), an Implantable Leadless Pacer (ILP), an Implantable Leadless Pressure Sensor (ILPS) and an Implantable Cardiac Defibrillator (ICD), which contain sensors that collect physiological signals and transmit them as data to a physician device or to a remote server where they can be viewed. Further long-term trends of each of these data are visualized to help guide patient care. The signals are generally processed and presented independently from the other numerous signals or health data.

Further, there above state-of-the-art solutions provide numerous health data of the patient than can result in "information overload" for the patient or health care practitioner (HCP) and may not provide a holistic and clear picture of the patient's health condition. To derive a high number of health data by an implanted medical device additionally needs a lot of energy and reduces longevity of the respective device which should be avoided. Additionally, physiological data measured within or at a patient often do not show the full picture of the patient's well-being. That's why the present clinical practice provides medical consultations or check-ups for the patient, for example one to a few times per year (depending on the patient's disease) to receive additional information about the patient's health condition and to adapt therapy accordingly. However, the frequency of feedback and recommendations between a patient and their HCP often seems too limited.

It is therefore desirable to provide a more energy efficient and less complicated system and respective operating method which provide a more holistic picture on the health condition of a specific patient and gives the patient a feedback in a more timely manner.

The above problem is solved by a system for supporting a patient's health control comprising a medical device and an extracorporeally located remote device with the features of claim 1, by a method for operating such system with the features of claim 7, by a computer program product with the features of claim 13, and by a computer readable data carrier with the features of claim 14.

In particular, the problem is solved by a system for supporting a patient's health control comprising a medical device and an extracorporeally located remote device, wherein the system has a training state and an application state, wherein the remote device comprises an artificial intelligence module (AI module), wherein the remote device and the medical device are configured to be temporarily connected for data exchange using a communication connection, wherein in the application state the remote device or the medical device is configured to provide a suitable quality-of-life (QOL) recommendation to the patient, wherein the suitable QOL recommendation is determined based on at least one recently measured physiological data of the patient by the medical device and based on a QOL calculation specification provided by the AI module, wherein in the training state the AI module is configured to determine the QOL calculation specification based on a plurality of measured physiological data of the patient received from the medical device using the communication connection and a plurality of gathered QOL information received from the patient and to pair the physiological data of the patient and the QOL information based on their respective time stamp indicating the measurement time of the respective physiological data and the gathering time of the respective QOL information, respectively.

The above system determines and presents actionable QOL recommendations to the patient more frequently to improve the quality of life outcomes for the patient, such as sleep quality, energy level, or mood, self-esteem and pain in a more frequent way than previously provided by medical consultations or check-ups. The above system further realizes to limit energy consumption by the medical device because the determined QOL calculation specification is based on measured physiological data which are important and have a strong influence on the determined QOL calculation specification and thereby to the QOL outcome of the patient. Physiological data that are insignificant for this QOL calculation specification are not measured so that their determination does not consume any energy. Accordingly, the QOL calculation specification concentrates on significant physiological data.

Further the above system provides a connection of physiological data and QOL information, wherein QOL information or measures refer to the patient's reception of the physical and mental health. The QOL information is gathered, for example, using questions directed to the patient about such perceptions at the moment or during a time period addressed in the respective question. The medical device or the remote device may prompt the patient with various questions referring to the perceptions of the physical and mental health. The device may, for example, allow the patient to response with either multiple-choice answers or a rating answer. One example of a multiple-choice question/answer may be "How did you sleep?" with possible responses such as "Very Bad", "Bad", "OK", "Good", "Very Good". An example of a rating question/answer may be "How did you sleep?" with a rating response between 0 and 10. Other examples of questions include "How much energy do you have right now?", or "How is your mood right now?", or "How stressed are you right now?". Accordingly, the QOL information may comprise, for example, information on sleep quality, as perceived by the patient. Other examples of perceptions include mood, stress, self-esteem, and to some degree, pain. Such questions cover aspects of the health condition of the patient which cannot be identified directly by physiological measurements. The answers to above questions will be stored in a respective data memory of the respective device together with a timestamp which reflects the date and time at which the patients provided the respective QOL information.

In the above system the medical device may be, for example, a pacemaker, an ICM, an ILP, a defibrillator, an ILPS, or an ICD. The medical device may be an implantable medical device which is fully or at least partly implantable within the patient's body.

The medical device uses at least one sensor to collect one type or different types of physiological data. The signals collected from these various sensors can include, but are not limited to, ECG, impedance, activity, posture, heart sounds, blood pressure, oxygen saturation, blood sugar, respiration, etc. In one embodiment, the medical device may be connected to other devices (i.e. in a network) that provide additional sensor data. For example, the primary medical device may not have any blood sugar sensor, but can be connected (e.g. via Bluetooth) to a separate medical device that does have such blood sugar sensor. Physiological data may be any type of bodily parameter measurement value that may be derived by any type of sensor from the patient's body. Additionally, physiological data are also data which are not directly measurable but may be calculated from the measured data, for example a mean value of a pre-defined parameter value with regard to a pre-defined time period, e.g. the mean value per day. Such derived data are also referred to as "features" in the following.

The extracorporeally located remote device is a functional unit that can perform substantial computations, including numerous arithmetic operations and logic operations without human intervention, such as, for example, a personal mobile device (PMD), a smartphone, a handheld device, a desktop computer, a server computer, clusters/warehouse scale computer and/or embedded system. The remote device may be or may comprise, for example, a patient device (e.g. a smartphone) and/or a centralized remote device operated by a clinic or the HCP. In case, the remote device is a computer system, the remote device may comprise a relay or intermediate device, for example the patient device, and a central server, wherein the relay device transmits the determined physiological data and the gathered QOL information to the central computer facility. The patient device of the remote device may comprise an interface which may be used by the HCP or the patient to provide input data. If the remote device comprises several separate units, they are configured to communicate with each other using respective communication modules as described below.

At least a part of the at least one processor of the remote device is used for the algorithms forming the data analysis of the AI module. The AI module realizes an AI algorithm, wherein an algorithm is finite set of well-defined rules for the solution of the above problem in a finite number of steps or a sequence of operations for performing the above and below specific task. The AI algorithm may comprise at least one so-called machine learning algorithm where computers programs (algorithms) learn associations of predictive power from examples input data. Machine learning is most simply the application of statistical models to data using computers. Machine learning uses a broader set of statistical techniques than those typically used in medicine. AI algorithms further comprise, for example, a linear regression algorithm, so-called deep learning algorithms that are based on models with less assumptions about the underlying data and are therefore able to handle more complex data. Deep learning algorithms allow the AI module to be fed with large quantities of raw data and to discover the representations necessary for detection or classification. Deep learning algorithms rely on multiple layers of representation of the data with successive transformations that amplify aspects of the input that are important for discrimination and suppress irrelevant variations. Deep learning may be supervised or unsupervised. Such AI algorithm may further comprise supervised learning training computer algorithms to learn associations between inputs and outputs in data through analysis of outputs of interest defined by a (typically human) supervisor. Once associations have been learned based on existing data they can be used to predict future examples. AI algorithms may further comprise unsupervised learning computer algorithms that learn associations in data without external definition of associations of interest. Unsupervised learning is able to identify previously undiscovered predictors, as opposed to simply relying on known associations. AI algorithms further comprise reinforcement learning computer algorithms that learn actions based on their ability to maximize a defined reward. With regard to the present invention AI algorithms are used to process QOL information and physiological data, at least during the training state, to determine the QOL calculation specification representing strong dependencies or correlation of QOL information and physiological data which is used to provide suitable QOL recommendation to the patient. The above data processing may include, for example, a regression analysis and/or forming a decision tree ensemble.

In one embodiment, the AI module comprises a regression algorithm, a neural network with deep learning and/or a generative adversarial network and/or a self-organizing map. This means that embodiments of machine learning / AI approaches used by the AI module provided for preparation/training of the AI module prior analysis of physiological data and QOL information are:

- Neural networks with deep learning, i.e. neural networks with more than one hidden layer:

  - Feed Forward Networks with multiple hidden layers (well suited, since relatively easy to implement and train),
  - Convolutional Neuronal Networks, for example for applications in image, speech and biosignal recognition (i.e. multi-dimensional input vectors, also quite easy to handle), or
  - Recurrent Neuronal Networks (more challenging to train).

Alternatively may be used in the AI module:

- GANs (Generative Adversarial Networks) which are a good learning approach, since they may be trained with fewer training data sets (smaller patient population) and since the network can also be trained with "statistically" generated data, or
- Self organizing maps (such as the Kohonen feature map) may be used as tools for the automated identification of QOL calculation specification and are considered to be quite robust for this application.

The AI module comprises implemented/trained artificial intelligence, e.g. in form of above analysis, network or map and a respective analysis/assignment algorithm, wherein this artificial intelligence is provided/established prior starting with the analysis of the data. Alternatively, during analysis of the physiological data and QOL information in the application state, the physiological data and QOL information may be used for further improvement and/or training of the artificial intelligence, e.g. the analysis, network or map and algorithm. In the latter case, however, it may be necessary to implement a continuous quality assurance process in parallel to avoid an unwanted deviation of the artificial intelligence in an incorrect direction. This may be realized, for example, by regularly also performing the described analysis using the physiological data and QOL information of a training data set and automatically determining the output QOL calculation specification. In case the output QOL calculation specification using the improved AI module intelligence considerably deviates from the output QOL calculation specification assigned with the training data set the AI module is reset to a version referring to the directly preceding training state or any other preceding training state of the AI module.

In one embodiment, the training of the AI module is provided using physiological data and QOL information and may further be provided using health assessment information corresponding to respective personal and health data of a representative group of patients and/or physiological data and/or QOL information representing the specific patient for which the above system is to be used later for supporting his/her health control.

In a special preparation/training method of the AI module, the neural network may additionally be trained using physiological data/QOL information determined by scientific studies or models (disease-specific face-related visual biomarkers). Such training data may be generated, for example, according to physiological data of the disease of the patient under observation.

One embodiment of a QOL calculation specification comprises a calculation rule for which the kind of rule and the parameters of the calculation rule may be determined. Alternatively, the QOL calculation specification may comprise a decision tree ensemble. The QOL calculation specification assigns a suitable QOL recommendation, i.e. a recommendation for the patient or the HCP aimed at increasing the quality of life of the specific patient, to most recently measured physiological data for example by using at least one threshold which may form a part of the QOL calculation specification. The QOL recommendation may be the outcome of the QOL calculation specification based on the recently measured physiological data. For example, the QOL calculation specification comprises a plurality of QOL recommendations from which one is chosen based on the result of a calculation rule. The calculation rule may depend on data of at least one physiological measure (i.e. bodily parameter, e.g. heart rate, blood glucose value, blood pressure, etc.), wherein the AI algorithm determines the important measure of physiological data and does not consider any non-important measure. In one embodiment, the above mentioned calculation rule may assign/associate most recently measured physiological data to a QOL value as the result of the calculation rule. Dependent on the calculated QOL value, the suitable QOL recommendation to be provided to the patient may be chosen, wherein the possibility, that no QOL recommendation is given to the patient, is included. The QOL recommendation may be provided to the patient by a visually, audibly and/or tactile perceptible signal.

In the training state the whole system is trained by providing data, namely the AI module is configured to determine the QOL calculation specification based on a plurality of measured physiological data of the patient and/or similar patients and a plurality of gathered QOL information received from the patient and/or similar patients and to pair the physiological data of the patient and the QOL information based on their respective time stamp indicating the measurement time (i.e. date and clock time) of the respective physiological data and the gathering time (i.e. date and clock time) of the respective QOL information, respectively.

In contrast, the application state is a state of the system in which the determined QOL calculation specification is used to determine and provide a suitable QOL recommendation to the patient based on at least one recently measured physiological data of the patient by the medical device or, alternatively, by a remote device or part of it, wherein in this case the remote device receives the physiological data from the medical device using the communication connection between the medical device and the remote device.

At the end of the training state the QOL calculation specification is provided to the medical device or kept in the remote device to be used in the application state. Accordingly, the QOL calculation specification may run in the remote device and/or in the medical device. The QOL calculation specification assesses the physiological data determined by the medical device and provides a suitable QOL recommendation as an output of the QOL calculation specification. For example, it may provide an alert if a threshold defined in the QOL calculation specification is crossed. For example, if a strong correlation of the heart rate and the stress is detected and implemented in the QOL calculation specification, the output and QOL recommendation may be, for example "You seem stressed. Try taking a moment to relax." if the heart rate exceeds a threshold defined by the QOL calculation specification. Other examples may include a prompt that provides a specific recommendation based on the physiologic data, when appropriate. For example, if a strong relationship is found between mood and blood sugar levels, when the predicted mood crosses the threshold, the prompt may be "Not feeling great? Try having a snack".

In one embodiment of the system, the physiological data of the patient comprise a first physiological data of a first type and at least a second physiological data of a second type, wherein in the training state the AI module is configured to determine the QOL calculation specification by choosing a strong calculation rule from a plurality of possible calculation rules with regard to at least one specific type of physiological data of the first type and the at least one second type of physiological data. This means, that physiological data of different types may be included in the QOL calculation specification to enhance the reliability of the QOL calculation specification. Different physiological data type may be, for example, a first data type associated with energy level response (bed time, rise time, activity level throughout the day or within a pre-defined period of the day, ...), a second data type associated with sleep quality response (bed time, rise time, posture information during usual sleep time period, activity during usual sleep time period, ...), a third data type associated with mood (yelling, blood sugar level, blood pressure level, ), and a fourth data type associated with pain (heart rate variability, activity level, walking gait, posture, ...).

As indicated above, in one embodiment of the system, in the application state the QOL calculation specification calculates a QOL value based on the recently measured physiological data of the at least one specific type of physiological data and on the determined calculation rule, wherein the suitable QOL recommendation is chosen based on the fact whether the calculated QOL value crosses a pre-defined QOL threshold value or not, wherein the QOL threshold value may be automatically determined by the AI module or may be defined by the HCP or the patient, for example based on the calculation rule. Using a threshold value as a trigger (when it is crossed) for providing a QOL recommendation or choosing a specific one is an easy and fast determinable way of providing a QOL recommendation.

In one embodiment of the system, in the application state the AI module is configured to adapt the QOL calculation specification and/or the determined QOL threshold value based on the physiological data recently measured in the application state and QOL information recently gathered in the application state. Accordingly, the system may dynamically adapt to the health status of the patient and his/her quality of life, for example during progression of a chronic disease or after cure of a disease. For that, the AI module may request via the remote device QOL information and/or physiological data from the patient, for example at pre-defined time intervals. In case the QOL calculation specification runs at the remote device, the device may provide the received physiological data at pre-defined time intervals or continuously to the AI module. If the AI module determines that an adaption of the QOL calculation specification and/or QOL threshold is necessary, it provides the adaption within the remote device and/or communicates the adaption to the medical device.

In one embodiment of the system, in the application state the medical device is configured to measure at least one pre-defined physiological data and/or the remote device is configured to gather a pre-defined QOL information from the patient within a pre-defined time period after provision of one suitable QOL recommendation to the patient to assess the outcome of the QOL recommendation, the effect of the recommendation to the physiological data and the compliance of the patient. These data/information may be used to adapt the QOL calculation specification including, if applicable, the QOL threshold accordingly as indicated above.

Each of the remote device and the medical device may further comprise a data memory which may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device. In the data memory the measured and derived physiological data as well as QOL information and recommendation and QOL calculation specification (comprising, if applicable, at least one threshold) may be stored.

The communication connection between the remote device and the medical device may be provided, for example, by a receiver or transceiver module of the remote device forming a communication module of the remote device and a respective sender or transceiver module of the medical device forming a communication module of the medical device. The communication module of the remote device may establish a communication channel to the communication module of the medical device at pre-defined time intervals, on demand or continuously. The communication module of the respective medical or remote device provides (one-directional) data transmission to the remote computer facility, for example of physiological data and/or QOL information. In one embodiment data exchange may be bidirectional. Such communication may comprise communication over the air (i.e. wireless, without wire) and/or by wired media. The communication may use inductive magnetic means, acoustic methods (e.g. ultrasound), and/or acoustic, optical and/or electromagnetic waves, for example Bluetooth, WLAN, ZigBee, NFC, Wibree or WiMAX in the radio frequency region, Ethernet, or IrDA or free-space optical communication (FSO) in the infrared or optical frequency region.

Each of the remote device and the medical device comprises at least one processor which is regarded as a functional unit of the remote computer facility that interprets and executes instructions comprising an instruction control unit and an arithmetic and logic unit.

The above problem is further solved by a method of operating a system for supporting a patient's health control comprising a medical device and an extracorporeally located remote device, wherein the system has a training state and an application state, wherein the remote device comprises an artificial intelligence module (AI module), wherein the remote device and the medical device are temporarily connected for data exchange using a communication connection, wherein in the application state the remote device provides a suitable quality-of-life (QOL) recommendation to the patient based on at least one recently measured physiological data of the patient by the medical device and based on a QOL calculation specification provided by the AI module, wherein in the training state the QOL calculation specification is determined based on a plurality of measured physiological data of the patient received from the medical device using the communication connection and a plurality of gathered QOL information received from the patient, wherein the physiological data of the patient and the QOL information are paired based on their respective time stamp indicating the measurement time of the respective physiological data and the gathering time of the respective QOL information, respectively.

In one embodiment of the method, the physiological data of the patient comprise a first physiological data of a first type and at least a second physiological data of a second type, wherein in the training state the QOL calculation specification is determined by the AI module by choosing a strong calculation rule from a plurality of possible calculation rules with regard to at least one specific type of physiological data of the first type and the second type of physiological data.

In one embodiment of the method, in the application state the QOL calculation specification calculates a QOL value based on the recently measured physiological data of the at least one specific type of physiological data, wherein the suitable QOL recommendation is determined if the calculated QOL value crosses a QOL threshold value defined in the QOL calculation specification.

In one embodiment of the method, in the application state the QOL calculation specification and/or the pre-defined QOL threshold value is adapted based on the physiological data recently measured in the application state and QOL information recently gathered in the application state.

In one embodiment of the method, the remote device and/or the medical device runs the QOL calculation specification and/or wherein the medical device is an implantable medical device.

In one embodiment of the method, wherein in the application state the at least one pre-defined physiological data is measured and/or a pre-defined QOL information from the patient is gathered within a pre-defined time period after provision of one suitable QOL recommendation to the patient.

The embodiments described for the medical device, the remote device and the system apply to the above methods, as well, causing the analogous advantages. It is referred to above description of embodiments of the medical device, remote device and the system in this regard.

The above method is, for example, realized as a computer program (to be executed at or within the remote device and/or the medical device, in particular utilizing their processors) which is a combination of above and below specified (computer) instructions and data definitions that enable computer hardware or a communication system to perform computational or control functions and/or operations, or which is a syntactic unit that conforms to the rules of a particular programming language and that is composed of declarations and statements or instructions needed for an above and below specified function, task, or problem solution.

Furthermore, a computer program product is disclosed comprising instructions which, when executed by a processor, cause the processor to perform the steps of the above defined method. Accordingly, a computer readable data carrier storing such computer program product is described.

The present invention will now be described in further detail with reference to the accompanying schematic drawing, wherein

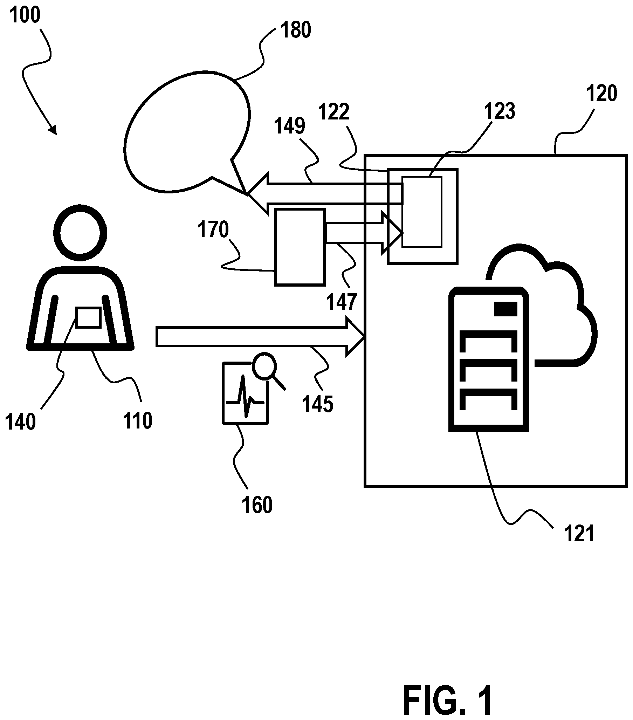

Fig. 1 shows one embodiment of a system for supporting a patient's health control

Fig. 1 shows one embodiment of a system 100 for supporting a patient's 110 health control comprising a remote device 120 having an AI module 121 within a centralized server and a patient device 122. Accordingly, the remote device 120 forms a computer facility having a system of several units/devices/modules connected with each other. The system 100 further comprises a medical device, for example a pacemaker 140, implanted within the patient 110. The patient 110 may have a chronic disease such as heart failure.

Generally, the system has a training state and an application state, wherein initially the system operates in the training state before it enters the application state. The system may re-enter the training state after some time of usage of the system in the application state.

In the training state and in the application state the pacemaker 140 regularly generates physiological data of at least one physiological measure (i.e. monitors at least one bodily parameter), for example provides regularly, for example twice a day, an ECG recording 160. The ECG recording 160 is an example of physiological data in the sense of the present invention. For ECG recording 160, the pacemaker 140 comprises a sensor measuring the electrical ECG signals (electrodes and respective hard- and software) of the patient's heart. During a training state of the system, at about the same time at which the ECG recording 160 of the patient 110 is measured, the patient is asked QOL questions using the patient device 122 to gather QOL information, for example, whether he/she feels well and whether the last night's sleep was well. The response of the patient 110 to these questions is the QOL information of the patient 110 represented by rectangle 170 in Fig. 1. The pacemaker 140 transmits the ECG data 160 to the remote device 120 (see arrow 145). Similarly, the gathered QOL information 170 provided by the patient 110 using a touch screen 123 of the patient device 122 (represented by arrow 147) are received by the remote device 120.

In the training state and in the application state, the pacemaker 140 may use its sensors to collect further types of physiological data and may send them to the remote device 120. The signals collected from these various sensors form further physiological data and may include, but are not limited to, impedance, activity, posture, heart sounds, blood pressure, oxygen saturation, blood sugar, respiration, etc. Additionally, the pacemaker 140 may be connected to other devices (i.e. in a network, not shown) that provide additional sensor data. For example, the pacemaker 140 may not have a blood sugar sensor, but may be connected (e.g. via Bluetooth) to a separate device that does have a blood sugar sensor (not shown in Fig. 1). This sensor provides blood sugar level data as further physiological data.

In the training state, after the physiological data such as ECG data 160 are collected, the pacemaker 140 may process the data to extract derived physiological data (so-called "features"). A simple example of a feature may be the average activity level throughout the day. A slightly more complicated example may be twenty-four different features, each of which represents the average activity level for each hour within the day. An even more complicated feature may be the time spent per day doing pre-defined activities, such as "walking", "running", "sleeping", "talking", "eating".

Other features may include, but are not limited to, resting heart rate, resting blood pressure, blood sugar levels, heart rate during activity, respiration rate, tidal volume, resting posture, etc. These features may be calculated over the average of a day, or averaged over every hour (essentially producing 24 features), or averaged over every 5 minutes, etc. Alternatively, the features might only be calculated at specific times, such as when patient feedback is gathered (as described more below). Other features may be the times of particular events in the day, including but not limited to, bed time (time the patient went to sleep), rise time (time the patient woke up from sleep to begin the next day), eating times, medication times, etc.

The calculation of such features may be provided by the processor of the pacemaker 140, and then the features will be transmitted to the remote device 120. Alternatively, the pacemaker 140 may not calculate any features, and may simply transmit the physiological data, such as the ECG 160, to the remote device 120 where the calculation of features will take place instead. This second approach has the advantage of reducing battery consumption on the pacemaker 140. Both approaches may also be combined, for example, calculating simple features (average activity level throughout the day) on the pacemaker 140, and calculating more complicated features (time spent in pre-defined activities) on the remote device 120.

The transmitted measured physiological data and derived physiological data are appropriately timestamped, depending on the type of feature. As an example, if the features are average resting heart rate per hour (resulting in 24 total features), each feature should be timestamped with the hour for which they were calculated.

Transmission of the physiological data (ECG 160) including calculated features to the remote device 120 may occur using any form of communication. Furthermore, when transmitting the data/features to the remote device 120, the patient device 122 may act as an intermediate device (e.g., the medical device 140 transmits physiological data and/or features to the patient device 122 via Bluetooth, and then the patient device relays this data to the central server of the remote device 120). Within the remote device 120 the ECG data 160 and, if applicable, further received physiological data and features calculated therefrom are transmitted to the AI module 121 where they are automatically analysed based on an AI algorithm as explained below in detail.

All physiological data and/or calculated features are received at the remote device 120. In one embodiment, the physiological data may pass through any number of devices acting as intermediate devices, but all data and features in the end arrive at a central server of the remote device 120 and its AI module 121 in order for the AI training to occur.

During the training state, the patient device 122 may prompt the patient 110 with various questions throughout the day. The patient device 122 may allow the patient 110 to respond with either multiple-choice answers or a rating answer. One example of a multiple-choice question/answer may be "How did you sleep?" with possible responses such as "Very Bad", "Bad", "OK", "Good", "Very Good". An example of a rating question/answer is described above. The responses to such questions form the QOL information 170 and will be stored with timestamps and sent to the same central server of the remote device 120 that the physiological data/calculated features of the medical device 140 were sent to.

The use of timestamps for these responses allows pairing of the responses with the physiological data (e.g. ECG data 160)/features described earlier. Thus, if the response for "How stressed are you right now?" was given at 3:30PM, this response may be paired with the feature "average resting heart rate between 3:25PM-3:35PM" derived from the ECG data 160, for example.

In the training state which may last, for example, a week or longer, sufficient physiological data (e.g. ECG data 160) and QOL information 170 (patient feedback) is collected to train the AI algorithm provided by the AI module 121 that learns the relationships between the physiological data (such as ECG data 160) and the QOL information 170 (patient's responses). During this initial training state (i.e. learning period), no QOL recommendations will be provided to the patient 110. The AI module 121, for example, provides a neural network with deep learning or linear regression, as described above, to the physiological data and the QOL information 170.

After a sufficient learning period, there will be enough QOL information 170 that are paired with the physiological data. For example, the AI algorithm may find that low resting heart rates are associated with low stress levels, while high rest heart rates are associated with high stress levels for this patient. While the above was a simple example, the true power AI algorithms are when many different types of physiological data from different physiological measures and different types of QOL information are involved.

After the AI algorithm has learned the association between the QOL information 170 and the physiological data, the learned associations are used to provide QOL recommendations 180. However, in order to improve specificity of the QOL recommendations 180, and to prevent excessive drain of the battery on the medical device 140, the following inventive solution may be used.

The AI module 121 determines which associations between physiological data and QOL information are important. For example, if many types of questions are asked/many types of QOL information received, and many types of physiological data are used, it may be that only a few associations are actually strong. This can be found (if the AI algorithm is a linear regression) by looking at normalized regression coefficients between the features and responses, or p-values between the features and responses. Thus, it may be found that out of ten different QOL information types and twenty different physiological data types, the only strong relationship found is between one QOL information and two physiological data types. If the AI algorithm is different, different methods for assessment of associations with regard to their strength may be used.

For example, consider if the system asked only two questions receiving responses with regard to two QOL information types (energy level and mood) and measured only three physiological data types. Furthermore, consider that the AI algorithm provided by the AI module 121 is linear regression. Therefore, the system would perform two linear regressions (one per question), with each regression incorporating all three physiological data types. The data may be normalized prior to the regression according to common practice. Standard output from linear regression includes the regression coefficients for each normalized feature, and the p-value for each of the features. If a p-value for a feature is very small ($<0.05$), it indicates that there is strong evidence that the feature is associated with the question/response. If it is large, it indicates that there is little evidence that the feature is associated with the question/response.

Therefore, if the linear regression for the QOL information with regard to the energy-level has no physiological data with a significant p-value, the system may decide that there is no relationship between the energy level and the measured physiological data and will therefore not proceed providing recommendations about energy level. If the other linear regression for the mood question has only one feature with a significant p-value, the system may decide that only this feature is needed to provide recommendations about mood.

In another example, the AI algorithm may be a decision tree ensemble instead of linear regression. In this case, one decision tree ensemble would be generated to predict energy level, and another ensemble would be generated to predict mood. For each ensemble, the importance of physiological data can be calculated by using, for example, Gini Importance or Mean Decrease in Impurity (common techniques for calculating importance in tree ensembles). Then, similar to the example of linear regression above, the features with low importance may be assumed to have no strong association with energy level or mood. Finally, new decision tree ensembles would be generated using only the important physiological data.

Once the strong associations are found, the remote device 120 is configured to send a message to the medical device (pacemaker 140) regarding which physiologic data have the strong association. For example, if the only strong association is found between stress levels and resting heart rate, the pacemaker will be informed to determine the resting heart rate.

Furthermore, the remote device 120 will send the determined QOL calculation specification to the pacemaker 140 and/or to the patient device 122 representing the found association comprising a determined relationship and a threshold for providing a respective QOL recommendation. For example, if the relationship between stress level (on a scale of 0-10) and resting heart rate is found to be

$$Stress\ Level = \frac{HeartRate}{10} - 5$$

then this equation shall be sent to the pacemaker 140 and/or the patient device 122. If a decision tree ensemble was used instead of linear regression, then the ensemble may be sent to the pacemaker 140 or the patient device 122 (which would manifest as a series of if-else statements).

The QOL calculation specification further comprises a threshold to generate an alert (i.e. a QOL recommendation) based on the above relationship which is sent from the remote device 120 to the patient device 122 or the medical device 120. This threshold may be set automatically based on the type of QOL information (e.g. always have a threshold of 5 for stress levels), or the patient device 122 may be triggered to set the threshold by the patient via the patient device 122 based on the above determined relationship. In the present example regarding resting heart rate and stress level, the threshold value may be 5 of maximum 10 for the stress level.

Once the important physiological data, the relationship between the physiological data and the QOL information as well as the at least one threshold is known by the pacemaker 140 and the patient device 122, the system switches into the application state and the pacemaker 140 measures the important physiological data (heart rate) throughout the day and calculates the predicted QOL information (also referred to as QOL value) in real-time, and provide an alert (QOL recommendation 180) if the QOL value crosses the threshold (in this example: 5). Continuing with the example above of stress level and resting heart rate, the device would therefore determine resting heart rate throughout the day (for example in 10 minutes windows), calculate the stress level based on the equation above, and then provide an alert (QOL recommendation) if the stress level crosses the threshold (represented by arrow 149 in Fig. 1 showing the provision of the QOL recommendation 180), for example by showing the alert at the touch screen 123.

The alert/recommendation 180 may be provided by the patient device 122 or sent by the pacemaker 140 to it, which then prompts the patient based on the type of alert. For example, if the alert is based on stress levels, the prompt may be "You seem stressed. Try taking a moment to relax" on the touch screen 123. The prompt may be emphasized by a respective acoustic signal causing an alert of the patient. Other examples may include a prompt that provides a specific recommendation based on the physiologic data, when appropriate.

Note that the above method and device has several advantages. By using only the most important associations, the medical device does not waste resources collecting data from useless physiologic measures. Furthermore, by calculating the predicted QOL recommendation on the medical device, it also saves resources by not continuously transmitting data to the remote device 120 where the calculation would otherwise have to occur.

As described above, after the initial training state, the system may begin providing QOL recommendations in the application state. However, the learning does not need to end at this point. The system may still continue to collect various physiologic data and ask the patient for feedback to receive QOL information during the application state, as well, and thereby learn more associations, as some associations make take more data and time. The continuous collection of physiological data and QOL information may also be used to improve already-found associations, as more data will always improve the predictive power of the QOL calculation specification provided by the AI algorithm.

To further improve the associations, the QOL recommendations described above may begin with a question. Therefore, instead of the recommendation: "You seem stressed. Try taking a moment to relax", the prompt may start with "Are you feeling stressed?". If the patient 110 responds "Yes", then it can continue and provide the recommendation "Try taking a moment to relax.". If the patient 110 responds "No", however, then this information can be used to adjust the QOL calculation specification regarding stress levels and resting heart rate, for example.

Furthermore, an additional question may be asked some time after the QOL recommendation was given to the patient to see if the QOL recommendation was useful. For example, if the system determines an association between mood and blood sugar levels, and recommends that the patient eat a snack due to improve their mood, the system may follow-up some after the recommendation with a prompt such as "Did the snack help?". If the patient responds "Yes", the system can further reinforce the association between the patient's mood and blood sugar. If the patient responds "No", however, this QOL information can be used to adjust the QOL calculation specification between stress levels and blood sugar.

In one embodiment, the remote device 120 may also monitor compliance with the QOL recommendation in certain scenario. Take, for example, the scenario where a strong relationship is found between mood and blood sugar levels. If the system predicts that the patient's mood has decreased due to a decrease in blood sugar levels, and makes the recommendation that a patient has a snack, the medical device may monitor the blood sugar levels after provision of the QOL recommendation to see if the patient 110 has complied with this recommendation. For example, if the medical device does not detect an increase in the blood sugar level, or does not detect that the patient has eaten a snack using any other means (e.g. an advanced feature using accelerometer data), it may follow-up with another prompt such as "Did you eat your snack?" to remind them to follow the QOL recommendation.

**Claims**

1. A system (100) for supporting a patient's (110) health control comprising a medical device (140) and an extracorporeally located remote device (120), wherein the system has a training state and an application state, wherein the remote device (120) comprises an artificial intelligence module (AI module, 121), wherein the remote device (120) and the medical device (140) are configured to be temporarily connected for data exchange using a communication connection, wherein in the application state the medical device (140) or the remote device (120) is configured to provide a suitable quality-of-life (QOL) recommendation (180) to the patient (110), wherein the suitable QOL recommendation (180) is determined based on at least one recently measured physiological data (160) of the patient by the medical device (140) and based on a QOL calculation specification provided by the AI module (121), wherein in the training state the AI module (121) is configured to determine the QOL calculation specification based on a plurality of measured/determined physiological data (160) of the patient (110) received from the medical device (140) using the communication connection and a plurality of gathered QOL information (170) received from the patient (110) and to pair the physiological data (160) of the patient (110) and the QOL information (170) based on their respective time stamp indicating the measurement time of the respective physiological data (160) and the gathering time of the respective QOL information (170), respectively.

2. The system of claim 1, wherein the physiological data (160) of the patient comprise a first physiological data of a first type and at least a second physiological data of a second type, wherein in the training state the AI module (121) is configured to determine the QOL calculation specification by choosing a strong calculation rule from a plurality of possible calculation rules, for example with regard to at least one specific type of physiological data (160) of the first type and the at least one second type of physiological data.

3. The system of any one of the preceding claims, wherein in the application state the QOL calculation specification calculates a QOL value based on the recently measured physiological data (160) of the at least one specific type of physiological data, wherein the suitable QOL recommendation (180) is determined if the calculated QOL value crosses a QOL threshold value defined in the QOL calculation specification.

4. The system of any one of the preceding claims, wherein in the application state the AI module (121) is configured to adapt the QOL calculation specification and/or the pre-defined QOL threshold value based on the physiological data (160) recently measured in the application state and QOL information (170) recently gathered in the application state.

5. The system of any one of the preceding claims, wherein the remote device (120) and/or the medical device (140) is adapted to run the QOL calculation specification and/or wherein the medical device (140) is an implantable medical device.

6. The system of any one of the preceding claims, wherein in the application state the medical device (140) is configured to measure at least one pre-defined physiological data (160) and/or the remote device (120) is configured to gather a QOL information (170) from the patient (110) within a pre-defined time period after provision of one suitable QOL recommendation (180) to the patient.

7. A method of operating a system (100) for supporting a patient's (110) health control comprising a medical device (140) and an extracorporeally located remote device (120), wherein the system has a training state and an application state, wherein the remote device (120) comprises an artificial intelligence module (AI module, 121), wherein the remote device (120) and the medical device (140) are temporarily connected for data exchange using a communication connection, wherein in the application state the remote device (120) or the medical device (140) provides a suitable quality-of-life (QOL) recommendation (180) to the patient based on at least one recently measured physiological data (160) of the patient by medical device (140) and based on a QOL calculation specification provided by the AI module (121), wherein in the training state the QOL calculation specification is determined based on a plurality of measured physiological data (160) of the patient (110) received from the medical device (140) using the communication connection and a plurality of gathered QOL information (170) of the patient (110), wherein the physiological data (160) of the patient and the QOL information (170) are paired based on their respective time stamp indicating the measurement time of the respective physiological data and the gathering time of the respective QOL information, respectively.

8.   The method of claim 7, wherein the physiological data (160) of the patient (110) comprise a first physiological data of a first type and at least a second physiological data of a second type, wherein in the training state the QOL calculation specification is determined by the AI module (121) by choosing a strong calculation rule from a plurality of possible calculation rules, for example with regard to at least one specific type of physiological data (160) of the first type and the at least one second type of physiological data.

9.   The method of any one of the claims 7 to 8, wherein in the application state the QOL calculation specification calculates a QOL value based on the recently measured physiological data (160) of the at least one specific type of physiological data, wherein the suitable QOL recommendation (180) is determined if the calculated QOL value crosses a pre-defined QOL threshold value.

10.  The method of any one of the claims 7 to 9, wherein in the application state the QOL calculation specification and/or the pre-defined QOL threshold value is adapted based on the physiological data (160) recently measured in the application state and QOL information (170) recently gathered in the application state.

11.  The method of any one of the claims 7 to 10, wherein the remote device (120) and/or the medical device (140) runs the QOL calculation specification and/or wherein the medical device (140) is an implantable medical device.

12.  The method of any one of the claims 7 to 11, wherein in the application state the at least one pre-defined physiological data (160) is measured and/or a pre-defined QOL information (170) from the patient (110) is gathered within a pre-defined time period after provision of one suitable QOL recommendation (180) to the patient.

13.  A computer program product comprising instructions which, when executed by a processor, causes the processor to perform the steps of the method according to any one of the claims 7 to 12.

14.  Computer readable data carrier storing a computer program product according to claim 13.

**FIG. 1**

Europäisches
Patentamt

European
Patent Office

Office européen
des brevets

# EUROPEAN SEARCH REPORT

**Application Number**

EP 22 16 8430

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/251456 A1 (CONSTANTIN ALEXANDRA ELENA [US] ET AL) 15 August 2019 (2019-08-15) * paragraphs [0328], [0334], [0308], [0321], [0322], [0598], [0308] * | 1-14 | INV. G16H20/17 G16H50/30 |
| A | US 2021/338153 A1 (BARRERA OSVALDO ANDRES [US] ET AL) 4 November 2021 (2021-11-04) * paragraphs [0134], [0131], [0085], [0126]; figure 1 * | 1-14 | |
| A | US 10 770 182 B2 (CARDIAC PACEMAKERS INC [US]; BOSTON SCIENT SCIMED INC [US]) 8 September 2020 (2020-09-08) * the whole document * | 1-14 | |
| A | US 2021/229199 A1 (COLOMBO DARIO [IT] ET AL) 29 July 2021 (2021-07-29) * the whole document * | 1-14 | |
| A | US 2020/037942 A1 (HOWARD NEWTON [US]) 6 February 2020 (2020-02-06) * the whole document * | 1-14 | TECHNICAL FIELDS SEARCHED (IPC)  G16H |

The present search report has been drawn up for all claims

| Place of search | Date of completion of the search | Examiner |
|---|---|---|
| Munich | 27 September 2022 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 8430

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2022

| Patent document cited in search report | | Publication date | Patent family member(s) | | Publication date |
|---|---|---|---|---|---|
| US 2019251456 | A1 | 15-08-2019 | AU | 2019217879 A1 | 23-07-2020 |
| | | | CA | 3089642 A1 | 15-08-2019 |
| | | | CN | 111655128 A | 11-09-2020 |
| | | | EP | 3749183 A1 | 16-12-2020 |
| | | | JP | 2021513136 A | 20-05-2021 |
| | | | US | 2019246914 A1 | 15-08-2019 |
| | | | US | 2019246973 A1 | 15-08-2019 |
| | | | US | 2019251456 A1 | 15-08-2019 |
| | | | US | 2019252079 A1 | 15-08-2019 |
| | | | WO | 2019157102 A1 | 15-08-2019 |
| US 2021338153 | A1 | 04-11-2021 | US | 2021338153 A1 | 04-11-2021 |
| | | | WO | 2021222514 A1 | 04-11-2021 |
| US 10770182 | B2 | 08-09-2020 | CN | 110769742 A | 07-02-2020 |
| | | | EP | 3624678 A1 | 25-03-2020 |
| | | | JP | 6868126 B2 | 12-05-2021 |
| | | | JP | 2020521128 A | 16-07-2020 |
| | | | US | 2018336970 A1 | 22-11-2018 |
| | | | WO | 2018213564 A1 | 22-11-2018 |
| US 2021229199 | A1 | 29-07-2021 | CN | 112236261 A | 15-01-2021 |
| | | | EP | 3639969 A1 | 22-04-2020 |
| | | | JP | 2022505311 A | 14-01-2022 |
| | | | KR | 20210074238 A | 21-06-2021 |
| | | | US | 2021229199 A1 | 29-07-2021 |
| | | | WO | 2020079645 A1 | 23-04-2020 |
| US 2020037942 | A1 | 06-02-2020 | NONE | | |

EPO FORM P0459

For more details about this annex : see Official Journal of the European Patent Office, No. 12/82